# EUROPEAN PATENT SPECIFICATION

(11) **EP 3 793 620 B1**
(45) Date of publication and mention of the grant of the patent: **12.07.2023**
(21) Application number: 19729123.0
(22) Date of filing: 17.05.2019
(51) Int. Cl.: A61K 51/12, A61M 5/14, A61M 5/142, A61M 5/145, A61M 5/168, A61M 5/178, A61M 5/24, A61M 25/00, A61N 5/10, G16H 40/63, G16H 40/20, G16H 20/40, A61M 25/01, A61M 39/10, G21G 1/06, G21G 4/08, G21H 5/02, A61M 5/20, A61B 18/00, A61M 5/315

(54) **MICROSPHERES CONTAINING RADIOACTIVE ISOTOPES AND OTHER MARKERS, AND ASSOCIATED METHODS**
MIKROKUGELN MIT RADIOAKTIVEN ISOTOPEN UND ANDEREN MARKERN SOWIE ZUGEHÖRIGE VERFAHREN
MICROSPHÈRES CONTENANT DES ISOTOPES RADIOACTIFS ET D'AUTRES MARQUEURS, ET PROCÉDÉS ASSOCIÉS

(30) Priority: 18.05.2018 US 201862673632 P; 18.05.2018 US 201862673628 P
(43) Date of publication of application: 24.03.2021
(73) Proprietor: Bard Peripheral Vascular, Inc., Franklin Lakes, NJ 07417 (US)
(72) Inventor: DROBNIK, Christopher D., Franklin Lakes, New Jersey 07417 (US); CRUSE, Terry, Franklin Lakes, New Jersey 07417 (US)
(74) Representative: Hoffmann Eitle
(86) International application number: PCT/US2019/032986
(87) International publication number: WO 2019/222700

(56) References cited:
- WO-A1-2018/154470
- US-A1- 2007 141 339
- US-A1- 2009 092 677
- US-A1- 2015 285 282

## Description

### TECHNICAL FIELD

This disclosure relates generally to radioembolization microspheres and to methods for preparing the microspheres and, more particularly, to multi-phase microspheres for radioembolization therapy and to microfluidic methods for preparing the microspheres.

### BACKGROUND

Embolization therapy is a minimally invasive surgery performed by interventional radiologists. Typical treatments may include entering the vasculature via a minor incision, such as in the arm or leg, and gaining access to the treatment site by use of guidewires and catheters, optionally aided by imaging techniques such as fluoroscopy. The embolic agent at the treatment site embolizes the vessel, blocking off the flow of blood to tumors downstream from the treatment site and resulting in necrosis and/or shrinkage of the tumors.

Radioactivity may be added to embolization therapies by including a radioactive material in the embolic agent. Transarterial radioembolization, for example, is a transcatheter intra-arterial procedure commonly employed for the treatment of malignant tumors. During this procedure, a microcatheter is navigated into a patient's liver where radioembolizing microspheres loaded with a radioactive compound, such as yttrium-90 (⁹⁰Y), are delivered to the targeted tumors. The microspheres embolize blood vessels that supply the tumors while also delivering radiation to kill tumor cells. Commonly, the microspheres are solid or porous glass or polymeric spheres containing ytttium-89 that is converted by neutron irradiation to yttrium-90 or cation exchange resins onto which yttrium-90 is directly loaded.

Radioembolizing microspheres present numerous challenges in their preparation and handling, particularly where reproducibility and consistent reliability is required in therapeutic methods. For example, radioisotopes such as yttrium-90 can leach out of the microspheres over time, leading to inconsistent radiation dosing at the tumor site. Microspheres are challenging to produce with constant diameters and often require complex sieving processes to sort them by size. Microspheres can be more dense than fluid media such as water, physiological saline, or blood and, therefore, are prone to settling. In general, the production of microspheres may require expensive, specialized equipment and time-consuming processes to satisfy custom requirements from a medical practitioner, all with a constant concern of exposing those preparing the microspheres to harmful radiation.

Therefore, ongoing needs exist for embolic agents and preparation methods that enable greater efficiency and reproducibility in preparation of the embolic agents and that address some or all of the previously identified challenges.

US 2009/092677 A1 relates to compositions containing injectable particles in which the injectable particles contain at least two polymeric components that differ in composition from one another (e.g. because at least on polymeric component contains a polymer that is not present in another polymeric component).

US 2007/0141339 A1 describes block copolymer particles comprising a block copolymer including at least one first block having a glass transition temperature of at most 37°C and at least one second block having a glass transition temperature of greater than 37°C, wherein the particle has a diameter of less than about 100 microns, from about 300 microns to about 500 microns, from about 700 microns to about 900 microns, or from about 1,000 microns to about 1,200 microns.

US 2015/0285282 A1 is concerned with a method of making multiple emulsions comprising forming a first droplet from a first fluid stream surrounded by a second fluid while the second fluid is surrounded by a third fluid.

### SUMMARY

The invention is defined by the claims. Any subject-matter falling outside the scope of the claims is provided for information purposes only.

According to embodiments, a two-phase microsphere includes a primary phase and a first secondary phase surrounded by the primary phase. The primary phase includes a first resin. The first secondary phase includes a second resin and at least one of a radioactive isotope or a compound including at least one radioactive element. The two-phase microspheres may be formed by a microfluidic process.

According to embodiments, a three-phase microsphere includes a primary phase, a first secondary phase surrounded by the primary phase, and a second secondary phase surrounded by the primary phase and discrete from the first secondary phase. The primary phase includes a first resin. The first secondary phase includes a second resin and at least one of a radioactive isotope or a compound including at least one radioactive element. The second secondary phase includes a gas bubble. The three-phase microspheres may be formed by a microfluidic process.

These and other features, aspects, and advantages of the present invention will become better understood with reference to the following description and the appended claims.

Additional features and advantages of the embodiments described herein will be set forth in the detailed description which follows, and in part will be readily apparent to those skilled in the art from that description or recognized by practicing the embodiments described herein, including the detailed description which follows, the claims, as well as the appended drawings.

It is to be understood that both the foregoing general description and the following detailed description describe various embodiments and are intended to provide an overview or framework for understanding the nature and character of the claimed subject matter. The accompanying drawings are included to provide a further understanding of the various embodiments, and are incorporated into and constitute a part of this specification. The drawings illustrate the various embodiments described herein, and together with the description serve to explain the principles and operations of the claimed subject matter.

### BRIEF DESCRIPTION OF THE DRAWINGS

FIG. **1** is a two-phase microsphere according to embodiments of this disclosure.
FIG. **2** is a three-phase microsphere according to embodiments of this disclosure.
FIG. **3** is a schematic diagram of a microfluidic process for preparing two-phase microspheres according to embodiments of this disclosure.
FIG. **4** is a schematic diagram of a microfluidic process for preparing three-phase microspheres according to embodiments of this disclosure.

### DETAILED DESCRIPTION

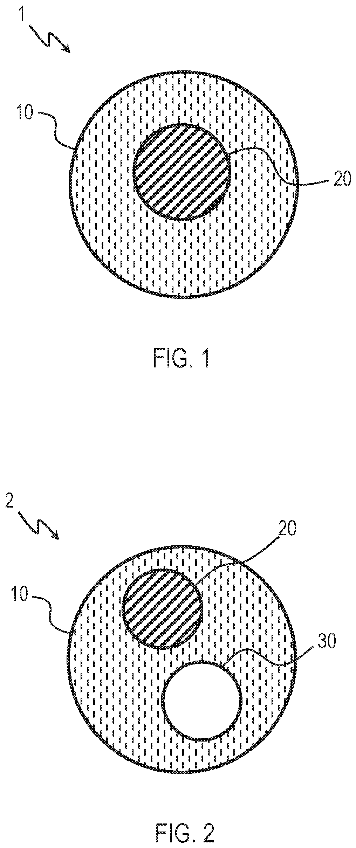

Reference will now be made in detail to embodiments of multiphase microspheres and, particularly, multiphase microspheres for radioembolization. As exemplary embodiments of multiphase microspheres, two-phase microspheres will be described with reference to FIG. 1, and three-phase microspheres will be described with reference to FIG. 2. It should be understood that microspheres having greater than three phases may be prepared as further embodiments by techniques analogous to those for preparing the two-phase microspheres and the three-phase microspheres. Methods for preparing the multiphase microspheres by a microfluidic process will be described subsequently.

Referring to FIG. 1, a two-phase microsphere **1** includes a primary phase **10** and a first secondary phase **20** surrounded by the primary phase **10.** The primary phase **10** includes a first resin. The first secondary phase **20** includes a second resin and at least one of a radioactive isotope or a compound including at least one radioactive element. In some embodiments of the two-phase microsphere **1,** the primary phase **10,** the first secondary phase 20, or both, may further include one or more additional compounds such as a therapeutic agent, a complex of a therapeutic agent, a fluorescent dye, a chelating agent, or a combination of any of these.

Referring to FIG. 2, a three-phase microsphere **2** includes a primary phase **10,** a first secondary phase **20** surrounded by the primary phase **10,** and a second secondary phase **30** surrounded by the primary phase **10** and discrete from the first secondary phase **20.** The primary phase **10** includes a first resin. The first secondary phase **20** includes a second resin and at least one of a radioactive isotope or a compound including at least one radioactive element. The second secondary phase **30** may be chosen from a third resin or a gas. In embodiments for which the second secondary phase **30** is a gas, the gas may be air, nitrogen, or any gas that is generally unreactive with the first resin and the second resin. The second secondary phase **30** may be present within the three-phase microsphere in an amount or volume fraction tailored to render the three-phase microsphere **2** neutrally buoyant in a chosen fluid such as water, physiological saline, blood, or any other carrier fluid suitable for administering embolic microbeads during a radioembolization procedure. In some embodiments of the three-phase microsphere **2,** the primary phase **10,** the first secondary phase **20,** the second secondary phase **30,** or any combination thereof, may further include one or more additional compounds such as a therapeutic agent, a complex of a therapeutic agent, a fluorescent dye, a chelating agent, or a combination of any of these.

In both the two-phase microsphere **1** and the three-phase microsphere **2,** the primary phase **10** forms the bulk volume of the microsphere that imparts an ability on the microsphere for the microsphere to embolize within a capillary during a radioembolization procedure. The secondary phase **20** of the two-phase microsphere (or the first secondary phase **20** of the three-phase microsphere) has a function of containing or encapsulating the radioactive isotope or compound of the microsphere in a manner that decreases the incidence and likelihood of leaching of radioactive material from the microsphere. In particular, the radioactive isotope or compound remains entrapped within the secondary phase **20,** while the primary phase **10** performs the embolic function. In contrast, in a single-phase microsphere, in which a radioactive compound is dispersed in a matrix of a single polymer or resin, some amount of radioactive compound is present out to the outer surface of the microsphere and, therefore, can escape or leach out from the microsphere. Leaching of this nature can lead to unpredictability and unreliability in radiation dosing at a tumor site, for example. The second secondary phase 30 of a three-phase microsphere 2 may be a gas or a third resin. When the second secondary phase 30 of the three-phase microsphere is a gas, such as air, for example, the inclusion of this phase, particularly with respect to a specific volume fraction, can adjust the overall density of the microbead as a whole. As the density of the microbead relates to its buoyancy in a liquid solution such as water, carrier, or blood, the inclusion of a gas phase can optimize flow characteristics of the microbead such as by preventing its settling to the bottom of the liquid solution. When the second secondary phase 30 of the three-phase microsphere is a third resin, the third resin may be selected, for example, to have particular compatibility with additional compounds that may be further included in the microbeads, such as therapeutic agents, for example.

In both the two-phase microsphere **1** and the three-phase microsphere **2,** the first resin and the second resin may be identical or different. As used herein, the term "resin" refers to any compound that can exist in an uncured form capable of flowed introduced in the uncured form through a microfluidics system, as will be described subsequently in greater detail, then cured by an additional process such as heating, UV irradiation, or other polymerization reaction, to form a solid, cured material. In some embodiments, the first resin, the second resin, or both, may be insoluble in water and/or physiological fluids such as blood, may be soluble in water and/or physiological fluids such as blood, may be bioresorbable, or may be biodegradable. In some embodiments, the first resin, the second resin, or both, may be water-swellable polymer materials. In some embodiments, the first resin, the second resin, or both, may be stable to gamma irradiation. In some embodiments, the first resin, the second resin, or both, may be impermeable to water. Exemplary compounds suitable as the first resin or the second resin will now be described.

Biodegradable and bioresorbable materials are materials that degrade and/or are reabsorbed safely within the body. Examples of biodegradable and bioresorbable materials may include, without limitation, polyglycolic acid (PGA), polyhydroxy butyrate (PHB), polyhydroxy butyrates-co-beta hydroxyl valerate (PHBV), polycaprolactone (PCL), Nylon-2-nylon-6, polylactic-polyglycolic acid copolymers, PLGA-polyethylene glycol (PEG)-PLGA (PLGA-PEG-PLGA), carboxymethylcellulose-chitosan (CMC-CCN), chitosan, hydroxyethyl acrylate (HEA), iron-based alloys, magnesium-based alloys, and combinations thereof.

Further examples of the first resin, the second resin, or both, include Poly(methylmethacrylate) (PMMA), sulfonated polystyrene-co-divinylbenzene (PSS-DVB), polylactic-co-glycolic acid (PLGA), PLGA-Polyethylene glycol (PEG)-PLGA (PLGA-PEG-PLGA), carboxymethylcellulose-chitosan (CMC-CCN), chitosan, hydroxyethyl acrylate (HEA), poly-4-hydroxybutyrate (P4HB), polyacrylamides and elastin-like proteins, CMC (oxidized carboxymethyl cellulose) / alginate / chitosan, methacrylated hyaluronic acid, cross-linked alginate based polymers (e.g. alginate and poly(N-isopropylacrylamide, alginate-PVA, CMC-alginate,alginate-PCL), functionalized poly(N-isopropylacrylamide), chitosan-alginate, copolymers of N-isopropylacrylamide(NIPAAm)-poly(ethylene glycol)-polycaprotactone-alginate, poly(N-vinylcaprolactam-co-glycidyl methacrylate), poly(N,N-dimethacrylamide-co-glycidyl methacrylate), acrylate based polymers, gelatin-PVA, polyacrylamide gels, polyvinyl alcohols, polyacrylic acids, polymethacrylic acids, poly vinyl sulfonates, carboxymethyl celluloses, hydroxyethyl celluloses, substituted celluloses, polyacrylamides, polyethylene glycols, polyamides, polyureas, polyurethanes, polyesters, polyethers, polystyrenes, polysaccharides, polylactic acids, polyethylenes, polymethylmethacrylates, polycaprolactones, polyglycolic acids, poly(lactic-co-glycolic) acids, and combinations thereof.

In various embodiments, the first resin, the second resin, or both may include a water-swellable polymer material that includes a natural hydrogel polymer such as a chitosan or a polysaccharide, or a synthetic hydrogel polymer such as a polyacrylate, a polyamide, a polyester, a polysaccharide, a poly(methylmethacrylate), or a poly(vinyl alcohol), for example. In some embodiments, the water-swellable polymer material may be biodegradable. Specific examples of water-swellable polymer materials include, without limitation, poly(4-hydroxybutyrate), methacrylated hyaluronic acids (hyaluronic acids being polymers of disaccharides composed of D-glucuronic acid and *N*-acetyl-D-glucosamine), chitosan-alginates, poly(*N*-isopropylacrylamide) copolymers, poly(*N*-isopropylacrylamide)-alginates, poly(*N*-isopropylacrylamide)-peptides, poly(*N*-isopropylacrylamide)-α-acryloyloxy-β,β-dimethyl-γ-butyrolactone-hydrophilic Jeffamine, or poly(*N*-isopropyl-acrylamide)-poly(ethylene glycol) diacrylate-pentaerythritol tetrakis(3-mercapto-propionate). The resins may include may include water-swellable polymer materials that include derivatives of any of the foregoing materials, or may include combinations of any of the foregoing materials or their derivatives.

Examples of biocompatible resins suitable as the first resin, the second resin, or both, include, without limitation, epoxy resins, polyether ether ketone resins, high-density polyethylenes, or combinations thereof.

Further examples of materials suitable as the first resin, the second resin or both, include polyvinyl alcohols, polyacrylic acids, polymethacrylic acids, poly vinyl sulfonates, carboxymethyl celluloses, hydroxyethyl celluloses, substituted celluloses, polyacrylamides, polyethylene glycols, polyamides, polyureas, polyurethanes, polyesters, polyethers, polystyrenes, polysaccharides, polylactic acids, polyethylenes, polyolefins, polypropylenes, polymethylmethacrylates, polycaprolactones, polyglycolic acids, poly(lactic-co-glycolic) acids (e.g., poly(d-lactic-co-glycolic) acids), polysulfones, polyethersulfones, polycarbonates, nylons, silicones, linear or crosslinked polysilicones, and copolymers or mixtures thereof. In some embodiments, one or both of the resins can be highly water insoluble, high molecular weight polymers. Examples of such a polymer is a high molecular weight polyvinyl alcohol (PVA) that has been acetylized.

Still further examples of materials suitable as the first resin, the second resin, or both, include poly(D,L-lactide), poly(D,L-lactide-co-glycolide), poly(L-lactide-co-glycolide), poly(L-lactide-co-glycolide-co-ε-caprolactone) and poly(D,L-lactide-co-ethylene glycol), poly(L-lactide-co-ethylene glycol), poly(D,L-lactide-bl-glycolide), poly(L-lactide-bl-glycolide), poly(D,L-lactide-bl-ethylene glycol), poly(L-lactide-bl-glycolide), poly(D,L-lactide-bl-lycolide-bl-caprolactone), poly(L-lactide-bl-glycolide-bl-ethylene glycol, and a poly(ester amide).

Still further examples of materials suitable as the first resin, the second resin, or both, include, without limitation, polycaprolactone, poly(L-lactide), poly(D,L-lactide), poly(D,L-lactide-co-PEG) block copolymers, poly(D,L-lactide-co-trimethylene carbonate), polyglycolide, poly(lactide-co-glycolide), polydioxanone (PDS), polyorthoester, polyanhydride, poly(glycolic acid-co-trimethylene carbonate), polyphosphoester, polyphosphoester urethane, poly(amino acids), polycyanoacrylates, poly(trimethylene carbonate), poly(iminocarbonate), polycarbonates, polyurethanes, copoly(ether-esters) (e.g. PEO/PLA), polyalkylene oxalates, polyphosphazenes, PHA-PEG, and combinations thereof. The PHA may include poly(α-hydroxyacids), poly(β-hydroxyacid) such as poly(3-hydroxybutyrate) (PHB), poly(3-hydroxybutyrate-co-valerate) (PHBV), poly(3-hydroxyproprionate) (PHP), poly(3-hydroxyhexanoate) (PHH), or poly(4-hydroxyacid) such as poly poly(4-hydroxybutyrate), poly(4-hydroxyvalerate), poly(4-hydroxyhexanoate), poly(hydroxyvalerate), poly(tyrosine carbonates), poly(tyrosine arylates), poly(ester amide), polyhydroxyalkanoates (PHA), poly(3-hydroxyalkanoates) such as poly(3-hydroxypropanoate), poly(3-hydroxybutyrate), poly(3-hydroxyvalerate), poly(3-hydroxyhexanoate), poly(3-hydroxyheptanoate) and poly(3-hydroxyoctanoate), poly(4-hydroxyalkanaote) such as poly(4-hydroxybutyrate), poly(4-hydroxyvalerate), poly(4-hydroxyhexanote), poly(4-hydroxyheptanoate), poly(4-hydroxyoctanoate) and copolymers including any of the 3-hydroxyalkanoate or 4-hydroxyalkanoate monomers described herein or blends thereof, polyglycolide, poly(D,L-lactide-co-glycolide), poly(L-lactide-co-glycolide), polycaprolactone, poly(lactide-co-caprolactone), poly(glycolide-co-caprolactone), poly(dioxanone), poly(ortho esters), poly(anhydrides), poly(tyrosine carbonates) and derivatives thereof, poly(tyrosine ester) and derivatives thereof, poly(imino carbonates), poly(glycolic acid-co-trimethylene carbonate), polyphosphoester, polyphosphoester urethane, poly(amino acids), polycyanoacrylates, poly(trimethylene carbonate), poly(iminocarbonate), polyphosphazenes, silicones, polyesters, polyolefins, polyisobutylene and ethylene-alphaolefin copolymers, acrylic polymers and copolymers, vinyl halide polymers and copolymers, such as polyvinyl chloride, polyvinyl ethers, such as polyvinyl methyl ether, polyvinylidene halides, such as polyvinylidene chloride, polyacrylonitrile, polyvinyl ketones, polyvinyl aromatics, such as polystyrene, polyvinyl esters, such as polyvinyl acetate, copolymers of vinyl monomers with each other and olefins, such as ethylene-methyl methacrylate copolymers, acrylonitrile-styrene copolymers, ABS resins, and ethylene-vinyl acetate copolymers, polyamides, such as Nylon 66 and polycaprolactam, alkyd resins, polycarbonates, polyoxymethylenes, polyimides, polyethers, poly(glyceryl sebacate), polypropylene fumarate), poly(n-butyl methacrylate), poly(sec-butyl methacrylate), poly(isobutyl methacrylate), poly(tert-butyl methacrylate), poly(n-propyl methacrylate), poly(isopropyl methacrylate), poly(ethyl methacrylate), poly(methyl methacrylate), epoxy resins, polyurethanes, rayon, rayon-triacetate, cellulose acetate, cellulose butyrate, cellulose acetate butyrate, cellophane, cellulose nitrate, cellulose propionate, cellulose ethers, carboxymethyl cellulose, polyethers such as poly(ethylene glycol) (PEG), copoly(ether-esters) (e.g. poly(ethylene oxide-co-lactic acid) (PEO/PLA)), polyalkylene oxides such as poly(ethylene oxide), polypropylene oxide), poly(ether ester), polyalkylene oxalates, phosphoryl choline containing polymer, choline, poly(aspirin), polymers and co-polymers of hydroxyl bearing monomers such as 2-hydroxyethyl methacrylate (HEMA), hydroxypropyl methacrylate (HPMA), hydroxypropylmethacrylamide, PEG acrylate (PEGA), PEG methacrylate, methacrylate polymers containing 2-methacryloyloxyethyl-phosphorylcholine (MPC) and n-vinyl pyrrolidone (VP), carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), poly(vinylidene fluoride)-PEG (PVDF-PEG), PLURONIC^{™} surfactants (polypropylene oxide-co-polyethylene glycol), poly(tetramethylene glycol), hydroxy functional poly(vinyl pyrrolidone), biomolecules such as collagen, chitosan, alginate, fibrin, fibrinogen, cellulose, starch, dextran, dextrin, hyaluronic acid, fragments and derivatives of hyaluronic acid, heparin, fragments and derivatives of heparin, glycosamino glycan (GAG), GAG derivatives, polysaccharide, elastin, elastin protein mimetics, or combinations thereof.

The two-phase microspheres or the three-phase microspheres according to embodiments may have diameters of a size suitable for radiotherapy or radioembolization medical treatment. In some embodiments, for which the microbeads are intended to become stuck in a capillary, without necessarily fully embolizing, so as to deliver therapeutic radiation at a target site, individual microspheres may have diameters of about 10 micrometers (µm) to about 80 µm, or about 20 µm to about 60 µm, or about 30 µm to about 50 µm, for example. In some embodiments, for which the microbeads are intended to be completely embolic ("bland beads") and include a therapeutic agent, individual microspheres may have diameters of about 30 micrometers (µm) to about 1500 µm. In other embodiments, the microspheres may have diameters of about 30 µm to about 1500 µm, about 30 µm to about 1000 µm, about 30 µm to about 500 µm, about 30 µm to about 100 µm, about 100 µm to about 1500 µm, about 100 µm to about 1000 µm, about 100 µm to about 500 µm, about 500 µm to about 1500 µm, about 500 µm to about 1000 µm, or about 1000 µm to about 1500 µm.

The two-phase microspheres or the three-phase microspheres according to embodiments may include from about 30% by weight to about 70% by weight, or from about 35% by weight to about 65% by weight, or from about 40% to about 60% by weight, or about 45% by weight to about 55% by weight, or about 50% to about 70% by weight resin, based on the total weight of the individual microspheres. In further embodiments, individual microspheres may include from about 30% by weight to about 70% by weight, or from about 35% by weight to about 65% by weight, or from about 40% to about 60% by weight, or about 45% by weight to about 55% by weight, or about 50% to about 70% by weight resin, based on the total weight of the individual microspheres, where the resin is a water-swellable polymer material.

The two-phase microspheres or the three-phase microspheres according to embodiments may be loaded with a therapeutic agent or with a complex of a therapeutic agent and a carrier. Individual drug-loaded microspheres may include one therapeutic agent or a plurality of therapeutic agents.

In embodiments, the therapeutic agent may be a hydrophilic therapeutic agent, a water-soluble therapeutic agent, or a therapeutic agent that has at least some solubility in an aqueous solution. In some embodiments, the therapeutic agent may be a chemotherapeutic agent having at least some efficacy for treating a disease such as cancer. In some embodiments, the therapeutic agent may be a chemotherapeutic agent having at least some efficacy for treating a cancer such as hepatocellular carcinoma, liver cancer, prostate cancer, or breast cancer. The therapeutic agent may have one or more chemical moieties or atomic centers having a positive or negative charge or affinity. Examples of specific therapeutic agents may include, without limitation, doxorubicin, sorafenib, vandetanib, nivolumab, ipilimumab, regorafenib, irinotecan, epirubicin, pirarubicin, 5-fluorouracil, cisplatin, floxuridine, mitomycin C, derivatives of any of the foregoing, prodrugs of any of the foregoing, therapeutically acceptable salts or crystalline forms of any of the foregoing, or combinations of any of the foregoing. Further examples of suitable therapeutic agents include, without limitation, pirarubicin, mitoxantrone, tepotecan, paclitaxel, carboplatin, pemetrexed, penistatin, pertuzumab, trastuzumab, and docetaxel.

In some embodiments, the therapeutic agent may generally surround the microspheres of the microbead material but lack of covalent chemical bonds between the therapeutic agent and the microbead material. Despite lacking covalent chemical bonds, the therapeutic agent and microbead material may have noncovalent intermolecular interactions such as ionic interactions or a van der Waals interaction. In some embodiments, the therapeutic agent of the drug-loaded microbead may generally surround the microbead material and lack covalent chemical bonds to the polymer backbone water-swellable polymer material, yet the therapeutic agent may be chemically bonded to a functional group of the water-swellable polymer material. In some embodiments, the therapeutic agent is not chemically bonded to the water-swellable polymer material at all.

The microspheres may include an amount of therapeutic agent that has a desired therapeutic effect or activity, based on the intended use for the microspheres. The amount of therapeutic agent in the individual drug-loaded microspheres may be adjusted through particular techniques involved during drug loading, such as loading time, loading temperature, or concentration of therapeutic agent in a loading solution, for example. The amount of therapeutic agent in the individual drug-loaded microspheres may be adjusted through synthetic techniques involved for synthesizing the microspheres themselves, such as through adjusting polymer molecular weights, degree of hydrogel crosslinking, polymer density, or polymer porosity of the water-swellable polymer material. For example, when doxorubicin is the therapeutic agent, the amount of drug loading in the drug-loaded microspheres may be adjusted with respect to the number of negative charges in the polymer backbone of the water-swellable polymer material.

In example embodiments, the microspheres may include from about 1% by weight to about 25% by weight, or from about 1% by weight to about 20% by weight, or from about 1% by weight to about 15% by weight, or from about 2% by weight to about 25% by weight, or from about 5% by weight to about 25% by weight, or from about 10% by weight to about 25% by weight therapeutic agent, based on the total weight of the individual microspheres.

In some embodiments, a drug-loaded microbead may include a complex of a carrier and a therapeutic agent. In the complex, the therapeutic agent may be chemically bonded to the carrier or may be associated with the carrier by a non-covalent means such as encapsulation or a van der Waals interaction. In embodiments, the complex may be embedded within the microbead material. In further embodiments, the complex may be embedded within the water-swellable polymer material. When the complex is embedded within the microbead material, the carrier may be chemically bonded to the microbead material while the therapeutic agent is not chemically bonded to the microbead material. Without intent to be bound by theory, it is believed that when the therapeutic agent is bonded or associated with the carrier but is not chemically bonded to the microbead material, the drug-loaded microspheres may be less susceptible to shrinking as a result of replacing water molecules with drug molecules during drug loading. Accordingly, the final size distribution of the drug-loaded microspheres may be controlled more readily by selecting appropriate microbead sizes before the therapeutic agent is loaded.

In embodiments in which the microbeads include a complex of the carrier and the therapeutic agent, the carrier may be any pharmaceutically-acceptable compound that can complex with or encapsulate the therapeutic agent. In some embodiments, the carrier may have charged chemical groups or chemical groups with dipole moments that interact with corresponding chemical groups of the therapeutic agent having an opposite charge or opposite dipole moment. If the carrier is a polymeric material, the carrier may be a different material from the first resin or the second resin. Non-limiting examples of suitable carriers include polysaccharides, liposomes, polymeric micelles, Pluronics, polycaprolactone-b-methoxy-PEG, poly(aspartic acid)-b-PEG, poly(benzyl-L-glutamate)-b-PEG, poly(D,L-lactide)-b-methoxy-PEG, poly(β-benzyl-L-asparate)-b-PEG). Non-limiting examples of polysaccharides include dextrans and dextran sulfates such as dextran sodium sulfate. In one example embodiment, the carrier may include a dextran sodium sulfate having a weight-average molecular weight of from about 40 kDa (kilodalton) to about 500 kDa, or from about 50 kDa to about 300 kDa, or from about 100 kDa to about 300 kDa, or about 100 kDa to about 200 kDa.

In example embodiments, the microspheres may include from about 1% by weight to about 40% by weight, or from about 1% by weight to about 30% by weight, or from about 1% by weight to about 25% by weight, or from about 1% by weight to about 20% by weight, or from about 5% by weight to about 40% by weight, or from about 10% by weight to about 40% by weight, or from about 20% by weight to about 40% by weight carrier, based on the total weight of the individual microbead.

The two-phase microspheres or the three-phase microspheres according to embodiments include a radioactive isotope or a compound including at least one radioactive element. The radioactive isotope or compound may be a radiotherapeutic agent having at least some efficacy for treating a disease such as cancer. In some embodiments, the radioactive isotope or compound may have at least some efficacy for treating a cancer such as hepatocellular carcinoma, cancer that has metastasized to the liver, prostate cancer, or breast cancer. The radioactive isotope or compound may include a radioisotope such as a beta-gamma emitter that or a gamma emitter that emits sufficient gamma radiation to enable imaging. Examples of specific radioactive isotopes include, without limitation, bismuth-213, boron-10, cesium-131, cesium-137, cobalt-60, dysprosium-165, erbium-169, holmium-166, iodine-125, iodine-131, iridium-192, iron-59, lead-212, lutetium-177, molybdenum-99, palladium-103, phosphorus-32, potassium-42, radium-223, rhenium-186, rhenium-188, samarium-153, selenium-75, sodium-24, strontium-89, technetium-99m, thorium-227, xenon-133, ytterbium-169, ytterbium-177, and yttrium-90. Some other examples include actinium-225, astatine-211, bismuth-213, carbon-11, nitrogen-13, oxygen-15, fluorine-18, cobalt-57, copper-64, copper-67, fluorine-18, gallium-67, gallium-68, germanium-68, indium-111, iodine-123, iodine-124, krypton-81m, rubidium-82, strontium-82, and thallium-201. The microspheres according to embodiments herein may include a compound including any of the foregoing radioactive isotopes. In some specific embodiments, the microspheres may include yttrium-90 or a compound including a yttrium-90 atom such as yttrium phosphate (⁹⁰YPO₄), yttrium sulfate (⁹⁰Y₂(SO₄)₃) or (⁸⁹Y⁹⁰Y(SO₄)₃), or yttrium carbonate (⁹⁰Y₂(CO₃)₃) or (⁸⁹Y⁹⁰Y(CO₃)₃).

In example embodiments, the microspheres include water. In example embodiments, the microspheres may have a low water content such as less than 1% by weight, or less than 0.5% by weight, or less than 0.1% by weight, or less than 0.05% (500 ppm) by weight, or less than 0.02% (200 ppm) by weight, or less than 0.01% (100 ppm) by weight, or less than 0.005 (50 ppm) by weight, or less than 0.002% (20 ppm) by weight, or less than 0.001% (10 ppm) by weight water, based on the total weight of the individual microspheres. Without intent to be bound by theory, it is believed that a low water content of the microbead increases the shelf-life and long-term stability of the microbead. Further, it is believed that water contents significantly greater than 1% by weight (such as 2%, 3%, 5%, or 10%, for example) based on the total weight of the microbead, may lead to decomposition or hydrolysis of the therapeutic agent, instability or breaking apart of the water-swellable polymer, or a combination of these, within a few days or even a few hours, such that the microbead cannot be used for embolization procedures, even if the microbead is rehydrated. It is believed that the shelf-life and long-term stability of having water contents significantly greater than 1% by weight are not sufficiently long to ensure viability of the therapeutic agent over the time period from manufacture of the microbead to use of the in an embolization procedure. It is believed that selection of the water-swellable polymer material may correlate with the ability for water to be removed from the microspheres by lyophilization or other drying technique or combination of drying techniques in an amount sufficient to prevent decomposition of the therapeutic agent.

A low water content of the microbead, as previously described, may be attained by drying techniques. In this regard, the microspheres may be dry or nearly dehydrated compositions of the microspheres containing the embedded therapeutic agent or the embedded complex of the therapeutic agent and the carrier. The microspheres may have a powder-like consistency. Accordingly, the microspheres may be made suitable for injection into a subject being treated by rehydrating the microspheres so that the microspheres may be suitable for embolization. Regardless, the microspheres may be provided in such a form that a physician needs to add only an aqueous solution such as water or physiologically buffered saline solution to the microspheres to prepare the microspheres for use in an embolization procedure.

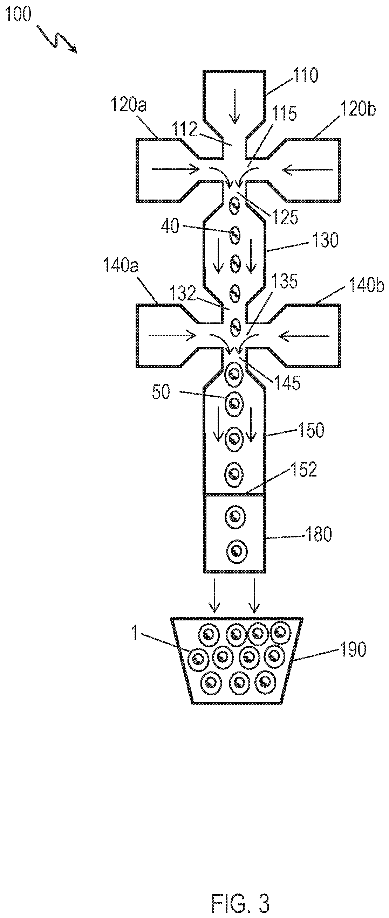

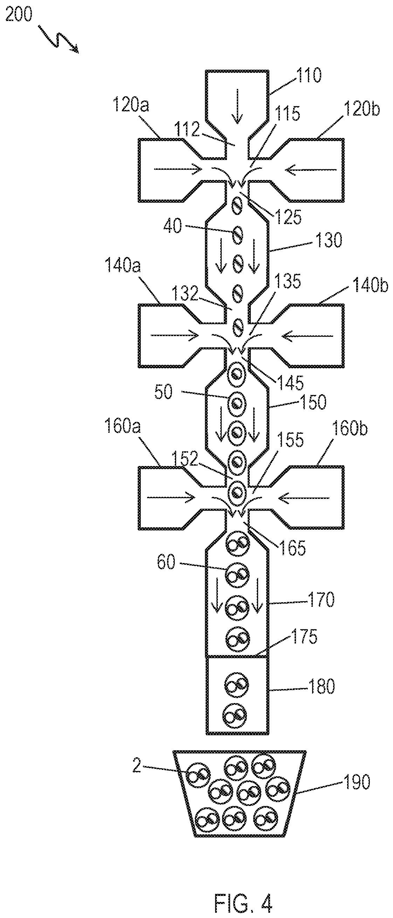

Multiphase microspheres have been described with respect to the embodiments of two-phase microspheres and three-phase microspheres. Methods for preparing the microspheres by microfluidic techniques will now be described with reference to FIGS. 3 and 4.

An example method of preparing two-phase microspheres **1** of FIG. 1 will be described with reference to FIG. 3. The two-phase microspheres **1** may be prepared in a two-tray microfluidic apparatus **100** by flowing a first fluid through a first longitudinal conduit **110** toward a first exit opening **112** of the first longitudinal conduit **110,** the first exit opening **112** being in fluidic communication with a first contact zone **115.** Simultaneously, a second fluid is flowed through a first transverse conduit that crosses the first longitudinal conduit **110** at the first contact zone **115.** In example embodiments, the second fluid may be flowed through the first transverse conduit **120a, 120b** from opposing directions toward the first contact zone **115.** The first transverse conduit **120a, 120b** is in fluidic communication with the first exit opening **112** and with a second exit opening **125** of the first transverse conduit **120a, 120b.** The second exit opening **125** is in fluidic communication with a second longitudinal conduit **130.** The flow of the first fluid enters the flow of the second fluid in a manner that produces a biphasic stream flowing into the second longitudinal conduit **130** toward a third exit opening **132** of the second longitudinal conduit **130.** The third exit opening **132** is in fluidic communication with a second contact zone **135.** The biphasic stream is composed of droplets **40** of the first fluid surrounded by a continuous phase of the second fluid.

The example method of preparing two-phase microspheres **1** further includes flowing a third fluid through a second transverse conduit **140a, 140b.** The second transverse conduit **140a, 140b** crosses the second longitudinal conduit **130** at the second contact zone **135** and is fluidic communication with the third exit opening **132** and with a fourth exit opening **145** of the second transverse conduit **140a, 140b.** The fourth exit opening **145** is in fluidic communication with a third longitudinal conduit **150.** The flow of the biphasic stream enters the flow of the third fluid in a manner that produces a triphasic stream flowing into the third longitudinal conduit **150** toward a fifth exit opening **152** of the third longitudinal conduit **150.** The triphasic stream includes two-phase droplets **50** surrounded by a continuous phase comprising the third fluid. The two-phase droplets **50** have an internal phase comprising the first fluid surrounded by an external phase comprising the second fluid. The triphasic flow including the two-phase droplets **50** then exits through the fifth exit opening **152.** The two-phase droplets **50** may be further processed in a processing vessel **180.** The processing vessel **180** may include means for curing materials in the two-phase droplets to form cured two-phase microspheres 1 that may be collected in a collecting vessel **190.** The processing vessel **180** further may include means for irradiating the two-phase droplets **50** such as by neutron irradiation, for example.

In the exemplary method, the first fluid may include an uncured first resin and at least one of a radioactive isotope or a compound that can be made radioactive upon neutron irradiation of the compound. The uncured first resin may be a compound that, upon curing, polymerizes or reacts to form a first resin, as previously described according to embodiments herein. The uncured first resin may be a compound that, upon curing, polymerizes or reacts to form a first resin that is biresorbable, biodegradable, or both. The uncured first resin may be a compound that, upon curing, polymerizes or reacts to form a first resin that is water-swellable. The radioactive isotope may be an isotope as previously described herein. An example of compounds that can be made radioactive upon neutron irradiation includes yttrium-89 chloride (⁸⁹YCl₃), which may be converted to yttrium-90 chloride (⁹⁰YCl₃) by neutron irradiation.

In the exemplary method, the second fluid includes an uncured second resin. The uncured second resin may be a compound that, upon curing, polymerizes or reacts to form a second resin, as previously described according to embodiments herein. In some embodiments, the second resin is identical to the first resin or is an identical polymer type that differs only in a physical characteristic such as molecular weight, for example. In other embodiments, the second resin is chemically compatible with the first resin but is a different chemical compounds.

The third fluid is a carrier fluid that is immiscible with the second fluid. Example carrier fluids include fluorocarbon oils.

An example method of preparing three-phase microspheres **2** of FIG. 2 will be described with reference to FIG. 4. The three-phase microspheres **2** may be prepared in a three-tray microfluidic apparatus **200** by flowing a first fluid through a first longitudinal conduit **110** toward a first exit opening 112 of the first longitudinal conduit **110,** the first exit opening **112** being in fluidic communication with a first contact zone **115.** Simultaneously, a second fluid is flowed through a first transverse conduit that crosses the first longitudinal conduit 110 at the first contact zone **115.** In example embodiments, the second fluid may be flowed through the first transverse conduit **120a, 120b** from opposing directions toward the first contact zone **115.** The first transverse conduit **120a, 120b** is in fluidic communication with the first exit opening **112** and with a second exit opening **125** of the first transverse conduit **120a, 120b.** The second exit opening **125** is in fluidic communication with a second longitudinal conduit **130.** The flow of the first fluid enters the flow of the second fluid in a manner that produces a biphasic stream flowing into the second longitudinal conduit **130** toward a third exit opening **132** of the second longitudinal conduit **130.** The third exit opening **132** is in fluidic communication with a second contact zone **135.** The biphasic stream is composed of droplets **40** of the first fluid surrounded by a continuous phase of the second fluid.

The example method of preparing three-phase microspheres **2** further includes flowing a third fluid through a second transverse conduit **140a, 140b.** The second transverse conduit **140a, 140b** crosses the second longitudinal conduit **130** at the second contact zone **135** and is fluidic communication with the third exit opening **132** and with a fourth exit opening **145** of the second transverse conduit **140a, 140b.** The fourth exit opening **145** is in fluidic communication with a third longitudinal conduit **150.** The flow of the biphasic stream enters the flow of the third fluid in a manner that produces a triphasic stream flowing into the third longitudinal conduit **150** toward a fifth exit opening **152** of the third longitudinal conduit **150.** The triphasic stream includes two-phase droplets **50** surrounded by a continuous phase comprising the third fluid. The two-phase droplets **50** have an internal phase comprising the first fluid surrounded by an external phase comprising the second fluid. The triphasic flow including the two-phase droplets **50** then exits through the fifth exit opening **152.**

The example method of preparing three-phase microspheres **2** further includes flowing a fourth fluid through a third transverse conduit **160a, 160b.** The third transverse conduit **160a, 160b** crosses the third longitudinal conduit **150** at the third contact zone **155** and is in fluidic communication with the fifth exit opening **150** and with a sixth exit opening **165** of the third transverse conduit **160a, 160b.** The sixth exit opening **165** is in fluidic communication with a fourth longitudinal conduit **170.** The flow of the biphasic stream enters the flow of the fourth fluid in a manner that produces a tetraphasic stream flowing into the fourth longitudinal conduit **170** toward a seventh exit opening **175** of the fourth longitudinal conduit **170.** The tetraphasic stream includes three-phase droplets **60** surrounded by a continuous phase comprising the fourth fluid. The three-phase droplets comprising a first internal phase comprising the first fluid, a second internal phase comprising the second fluid, and an external phase comprising the third fluid. In the three-phase droplets **60,** the first internal phase and the second internal phase are discrete from one another and are surrounded by the external phase. Thereupon, the three-phase droplets flow from the seventh exit opening **175** to a processing vessel **180.** The processing vessel **180** may include means for curing materials in the three-phase droplets to form cured three-phase microspheres **2** that may be collected in a collecting vessel **190.** The processing vessel **180** further may include means for irradiating the three-phase droplets **60** such as by neutron irradiation, for example.

In the exemplary method, the first fluid comprises a gas or an uncured first resin. The uncured first resin may be a compound that, upon curing, polymerizes or reacts to form a first resin, as previously described according to embodiments herein. The gas may be any gas that is unreactive with the first resin or the second resin, such as air or nitrogen, for example. The uncured first resin may be a compound that, upon curing, polymerizes or reacts to form a first resin that is biresorbable, biodegradable, or both. The uncured first resin may be a compound that, upon curing, polymerizes or reacts to form a first resin that is water-swellable. The second fluid comprises an uncured second resin. The uncured second resin may be a compound that, upon curing, polymerizes or reacts to form a second resin, as previously described according to embodiments herein. The uncured second resin may be a compound that, upon curing, polymerizes or reacts to form a second resin that is biresorbable, biodegradable, or both. The uncured second resin may be a compound that, upon curing, polymerizes or reacts to form a second resin that is water-swellable. In some embodiments, the second resin is identical to the first resin or is an identical polymer type that differs only in a physical characteristic such as molecular weight, for example. In other embodiments, the second resin is chemically compatible with the first resin but is a different chemical compounds.

At least one of the first fluid, the second fluid, or both, comprises a radioactive isotope or a compound that can be made radioactive upon neutron irradiation of the compound. The radioactive isotope may be an isotope as previously described herein. An example of compounds that can be made radioactive upon neutron irradiation includes yttrium-89 chloride (⁸⁹YCl₃), which may be converted to yttrium-90 chloride (⁹⁰YCl₃) by neutron irradiation.

The third fluid comprises an uncured third resin, which may be the same as or different from the uncured first resin, the uncured second resin, or both. The fourth fluid is a carrier fluid, such as a fluorocarbon oil, for example, that is immiscible with the third fluid.

In the exemplary methods for preparing either the two-phase microspheres **1** or the three-phase microspheres **2,** one or more of the first fluid, the second fluid, the third fluid, or the fourth fluid may further include a therapeutic agent, a complex of a therapeutic agent, a fluorescent dye, a chelating agent, a surfactant, a curing agent, a polymerization inhibitor, or a combination of any of these. Therapeutic agents have been described previously. Fluorescent dyes may include fluorescent compounds, for example, fluorescein. The dye compounds optionally may be derivatized with groups such as acrylic moieties that aid the incorporation of the dye into a resin. Chelating agents generally may ensure retention of the radioactive isotope within the first secondary phase of the multiphase microsphere, or may prevent leaching of the radioactive isotope into the primary phase of the multiphase microsphere. Surfactants, such as polysorbates, for example, stabilize droplets during the microfluidic process. Curing agents, such as ammonium persulfate, for example, enable curing of certain uncured resin materials on exposure thereof to heat. Other curing agents may enable UV curing or radiation curing. Polymerization inhibitors may avoid premature polymerization of the uncured resins during the microfluidic process, as some resins have a tendency to cure from exposure to the radioactivity of the radioactive isotope.

In the exemplary methods for preparing either the two-phase microspheres **1** or the three-phase microspheres **2,** the size of the microparticles, the relative amounts of first resin or gas, second resin, third resin, and radioactive isotope or compound may be controlled by adjusting process parameters such as flow rates or pressures of the first fluid, and/or the second fluid, and/or the third fluid, and/or the fourth fluid.

Unless otherwise defined, all technical and scientific terms used herein have the same meaning as commonly understood by one of ordinary skill in the art to which the invention belongs. The terminology used in the description herein is for describing particular embodiments only and is not intended to be limiting. As used in the specification and appended claims, the singular forms "a," "an," and "the" are intended to include the plural forms as well, unless the context clearly indicates otherwise.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

## Claims

1. A multiphase microsphere for radioembolization, the multiphase microsphere comprising:
a primary phase comprising a first resin; and
a first secondary phase surrounded by the primary phase, the first secondary phase comprising a second resin and at least one of a radioactive isotope or a compound including at least one radioactive element.

2. The multiphase microsphere of claim 1, wherein the first resin is a bioresorbable resin or a biodegradable resin; and/or wherein the first resin is a water-swellable polymer; and/or wherein the first resin and the second resin are identical.

3. The multiphase microsphere of any of the preceding claims, wherein the radioactive isotope comprises a beta emitting isotope or a gamma emitting isotope.

4. The multiphase microsphere of any of the preceding claims, wherein the multiphase microsphere further comprises a therapeutic agent; and/or
wherein the primary phase, the secondary phase, or both, further comprises a fluorescent dye.

5. The multiphase microsphere of any of the preceding claims, wherein the first resin is stable to gamma irradiation; and/or wherein the first resin is impermeable to water.

6. The multiphase microsphere of any of the preceding claims, further comprising a second secondary phase surrounded by the primary phase, the second secondary phase comprising a gas, optionally wherein the gas is air.

7. The multiphase microsphere of claim 6, wherein the multiphase microsphere is neutrally buoyant in water or is neutrally buoyant in human blood.

8. A method for forming two-phase microspheres, the method comprising:
flowing a first fluid through a first longitudinal conduit toward a first exit opening of the first longitudinal conduit, the first exit opening being in fluidic communication with a first contact zone;
flowing a second fluid through a first transverse conduit, the first transverse conduit crossing the first longitudinal conduit at the first contact zone and being in fluidic communication with the first exit opening and with a second exit opening of the first transverse conduit, the second exit opening being in fluidic communication with a second longitudinal conduit, whereby a biphasic stream flows into the second longitudinal conduit toward a third exit opening of the second longitudinal conduit, the third exit opening being in fluidic communication with a second contact zone, the biphasic stream comprising droplets of the first fluid surrounded by a continuous phase of the second fluid;
flowing a third fluid through a second transverse conduit, the second transverse conduit crossing the second longitudinal conduit at the second contact zone and being in fluidic communication with the third exit opening and with a fourth exit opening of the second transverse conduit, the fourth exit opening being in fluidic communication with a third longitudinal conduit, whereby a triphasic stream flows into the third longitudinal conduit toward a fifth exit opening of the third longitudinal conduit, the triphasic stream comprising two-phase droplets surrounded by a continuous phase comprising the third fluid, the two-phase droplets comprising an internal phase comprising the first fluid surrounded by an external phase comprising the second fluid; and
flowing the two-phase droplets from the fifth exit opening to a curing vessel; and
curing the two-phase droplets to form two-phase microspheres,
wherein:
the first fluid comprises an uncured first resin and at least one of a radioactive isotope or a compound that can be made radioactive upon neutron irradiation of the compound;
the second fluid comprises an uncured second resin;
the third fluid is immiscible with the second fluid.

9. A method for forming three-phase microspheres, the method comprising:
flowing a first fluid through a first longitudinal conduit toward a first exit opening of the first longitudinal conduit, the first exit opening being in fluidic communication with a first contact zone;
flowing a second fluid through a first transverse conduit, the first transverse conduit crossing the first longitudinal conduit at the first contact zone and being in fluidic communication with the first exit opening and with a second exit opening of the first transverse conduit, the second exit opening being in fluidic communication with a second longitudinal conduit, whereby a biphasic stream flows into the second longitudinal conduit toward a third exit opening of the second longitudinal conduit, the third exit opening being in fluidic communication with a second contact zone, the biphasic stream comprising droplets of the first fluid surrounded by a continuous phase of the second fluid;
flowing a third fluid through a second transverse conduit, the second transverse conduit crossing the second longitudinal conduit at the second contact zone and being in fluidic communication with the third exit opening and with a fourth exit opening of the second transverse conduit, the fourth exit opening being in fluidic communication with a third longitudinal conduit, whereby a triphasic stream flows into the third longitudinal conduit toward a fifth exit opening of the third longitudinal conduit, the fifth exit opening being in fluidic communication with a third contact zone, the triphasic stream comprising two-phase droplets surrounded by a continuous phase comprising the third fluid, the two-phase droplets comprising an internal phase comprising the first fluid surrounded by an external phase comprising the second fluid; and
flowing a fourth fluid through a third transverse conduit, the third transverse conduit crossing the third longitudinal conduit at the third contact zone and being in fluidic communication with the fifth exit opening and with a sixth exit opening of the third transverse conduit, the sixth exit opening being in fluidic communication with a fourth longitudinal conduit, whereby a tetraphasic stream flows into the fourth longitudinal conduit toward a seventh exit opening of the fourth longitudinal conduit, the tetraphasic stream comprising three-phase droplets surrounded by a continuous phase comprising the fourth fluid, the three-phase droplets comprising a first internal phase comprising the first fluid, a second internal phase comprising the second fluid, and an external phase comprising the third fluid, the first internal phase and the second internal phase being surrounded by the external phase; and
flowing the three-phase droplets from the seventh exit opening to a curing vessel; and
curing the three-phase droplets to form three-phase microspheres,
wherein:
the first fluid comprises a gas or an uncured first resin;
the second fluid comprises an uncured second resin;
at least one of the first fluid, the second fluid, or both, comprises a radioactive isotope or a compound that can be made radioactive upon neutron irradiation of the compound;
the third fluid comprises an uncured third resin; and
the fourth fluid is immiscible with the third fluid.

10. The method of claim 8 or 9, wherein the first resin is a bioresorbable resin or a biodegradable resin; and/or wherein the first resin is a water-swellable polymer; and/or wherein the first uncured resin and the second uncured resin are identical.

11. The method of claim 8 to 10, wherein the radioactive isotope comprises a beta emitting isotope or a gamma emitting isotope.

12. The method of any of claims 8 to 11, wherein the first fluid comprises the uncured first resin and a compound that can be made radioactive upon neutron irradiation of the compound, the method further comprising neutron irradiating the two-phase microspheres, optionally wherein the compound comprises yttrium-89 and the neutron irradiation converts the yttrium-89 to yttrium-90.

13. The method of any of the claims 8 to 12, wherein the first fluid, the second fluid, or both, further comprises a therapeutic agent; and/or
wherein the first fluid, the second fluid, or both, further comprises a fluorescent dye.

14. The method of any of the claims 9 to13, wherein the first fluid is a gas, optionally wherein the gas is air.

15. The method of any of claims 8 to 14, wherein the radioactive isotope comprises yttrium-90.

## Patentansprüche

1. Mehrphasen-Mikrokugel zur Radioembolisation, wobei die Mehrphasen-Mikrokugel umfasst:
eine primäre Phase, die ein erstes Harz umfasst; und
eine erste sekundäre Phase, die von der primären Phase umgeben ist, wobei die erste sekundäre Phase ein zweites Harz und mindestens eines aus einem radioaktiven Isotop oder einer Verbindung, die mindestens ein radioaktives Element beinhaltet, umfasst.

2. Mehrphasen-Mikrokugel nach Anspruch 1, wobei es sich bei dem ersten Harz um ein biologisch resorbierbares Harz oder ein biologisch abbaubares Harz handelt; und/oder wobei es sich bei dem ersten Harz um ein wasserquellbares Polymer handelt; und/oder wobei das erste Harz und das zweite Harz identisch sind.

3. Mehrphasen-Mikrokugel nach einem der vorstehenden Ansprüche, wobei das radioaktive Isotop ein beta-emittierendes Isotop oder ein gamma-emittierendes Isotop umfasst.

4. Mehrphasen-Mikrokugel nach einem der vorstehenden Ansprüche, wobei die Mehrphasen-Mikrokugel weiter ein therapeutisches Mittel umfasst; und/oder
wobei die primäre Phase, die sekundäre Phase, oder beide, weiter einen Fluoreszenzfarbstoff umfassen.

5. Mehrphasen-Mikrokugel nach einem der vorstehenden Ansprüche, wobei das erste Harz gegenüber Gammastrahlung stabil ist; und/oder wobei das erste Harz wasserundurchlässig ist.

6. Mehrphasen-Mikrokugel nach einem der vorstehenden Ansprüche, weiter eine zweite sekundäre Phase umfassend, die von der primären Phase umgeben ist, wobei die zweite sekundäre Phase ein Gas umfasst, wobei es sich bei dem Gas gegebenenfalls um Luft handelt.

7. Mehrphasen-Mikrokugel nach Anspruch 6, wobei die Mehrphasen-Mikrokugel in Wasser auftriebsneutral ist oder in menschlichem Blut auftriebsneutral ist.

8. Verfahren zum Bilden von Zweiphasen-Mikrokugeln, wobei das Verfahren umfasst:
Strömenlassen eines ersten Fluids durch einen ersten Längskanal in Richtung einer ersten Austrittsöffnung des ersten Längskanals, wobei die erste Austrittsöffnung mit einer ersten Kontaktzone in Fluidkommunikation steht;
Strömenlassen eines zweiten Fluids durch einen ersten Querkanal, wobei der erste Querkanal den ersten Längskanal an der ersten Kontaktzone kreuzt und mit der ersten Austrittsöffnung und mit einer zweiten Austrittsöffnung des ersten Querkanals in Fluidkommunikation steht, wobei die zweite Austrittsöffnung mit einem zweiten Längskanal in Fluidkommunikation steht, wodurch ein biphasischer Strom in den zweiten Längskanal in Richtung einer dritten Austrittsöffnung des zweiten Längskanals strömt, wobei die dritte Austrittsöffnung mit einer zweiten Kontaktzone in Fluidkommunikation steht, wobei der biphasische Strom Tröpfchen des ersten Fluids umfasst, die von einer kontinuierlichen Phase des zweiten Fluids umgeben sind;
Strömenlassen eines dritten Fluids durch einen zweiten Querkanal, wobei der zweite Querkanal den zweiten Längskanal an der zweiten Kontaktzone kreuzt und mit der dritten Austrittsöffnung und mit einer vierten Austrittsöffnung des zweiten Querkanals in Fluidkommunikation steht, wobei die vierte Austrittsöffnung mit einem dritten Längskanal in Fluidkommunikation steht, wodurch ein triphasischer Strom in den dritten Längskanal in Richtung einer fünften Austrittsöffnung des dritten Längskanals strömt, wobei der triphasische Strom Zweiphasen-Tröpfchen umfasst, die von einer kontinuierlichen Phase, die das dritte Fluid umfasst, umgeben sind, wobei die Zweiphasen-Tröpfchen eine innere Phase umfassen, die das erste Fluid umfasst, welche von einer äußeren Phase, die das zweite Fluid umfasst, umgeben sind; und
Strömenlassen der Zweiphasen-Tröpfchen von der fünften Austrittsöffnung zu einem Aushärtegefäß; und
Aushärten der Zweiphasen-Tröpfchen, um Zweiphasen-Mikrokugeln zu bilden,
wobei:
das erste Fluid ein ungehärtetes erstes Harz und mindestens eines aus einem radioaktiven Isotop oder einer Verbindung umfasst, die bei Neutronenbestrahlung der Verbindung radioaktiv gemacht werden kann;
das zweite Fluid ein ungehärtetes zweites Harz umfasst;
das dritte Fluid mit dem zweiten Fluid unmischbar ist.

9. Verfahren zum Bilden von Dreiphasen-Mikrokugeln, wobei das Verfahren umfasst:
Strömenlassen eines ersten Fluids durch einen ersten Längskanal in Richtung einer ersten Austrittsöffnung des ersten Längskanals, wobei die erste Austrittsöffnung mit einer ersten Kontaktzone in Fluidkommunikation steht;
Strömenlassen eines zweiten Fluids durch einen ersten Querkanal, wobei der erste Querkanal den ersten Längskanal an der ersten Kontaktzone kreuzt und mit der ersten Austrittsöffnung und mit einer zweiten Austrittsöffnung des ersten Querkanals in Fluidkommunikation steht, wobei die zweite Austrittsöffnung mit einem zweiten Längskanal in Fluidkommunikation steht, wodurch ein biphasischer Strom in den zweiten Längskanal in Richtung einer dritten Austrittsöffnung des zweiten Längskanals strömt, wobei die dritte Austrittsöffnung mit einer zweiten Kontaktzone in Fluidkommunikation steht, wobei der biphasische Strom Tröpfchen des ersten Fluids umfasst, die von einer kontinuierlichen Phase des zweiten Fluids umgeben sind;
Strömenlassen eines dritten Fluids durch einen zweiten Querkanal, wobei der zweite Querkanal den zweiten Längskanal an der zweiten Kontaktzone kreuzt und mit der dritten Austrittsöffnung und mit einer vierten Austrittsöffnung des zweiten Querkanals in Fluidkommunikation steht, wobei die vierte Austrittsöffnung mit einem dritten Längskanal in Fluidkommunikation steht, wodurch ein triphasischer Strom in den dritten Längskanal in Richtung einer fünften Austrittsöffnung des dritten Längskanals strömt, wobei die fünfte Austrittsöffnung mit einer dritten Kontaktzone in Fluidkommunikation steht, wobei der triphasische Strom Zweiphasen-Tröpfchen umfasst, die von einer kontinuierlichen Phase, die das dritte Fluid umfasst, umgeben sind, wobei die Zweiphasen-Tröpfchen eine innere Phase umfassen, die das erste Fluid umfasst, welche von einer äußeren Phase, die das zweite Fluid umfasst, umgeben sind; und
Strömenlassen eines vierten Fluids durch einen dritten Querkanal, wobei der dritte Querkanal den dritten Längskanal an der dritten Kontaktzone kreuzt und mit der fünften Austrittsöffnung und mit einer sechsten Austrittsöffnung des dritten Querkanals in Fluidkommunikation steht, wobei die sechste Austrittsöffnung mit einem vierten Längskanal in Fluidkommunikation steht, wodurch ein tetraphasischer Strom in den vierten Längskanal in Richtung einer siebten Austrittsöffnung des vierten Längskanals strömt, wobei der tetraphasische Strom Dreiphasen-Tröpfchen umfasst, die von einer kontinuierlichen Phase, die das vierte Fluid umfasst, umgeben sind, wobei die Dreiphasen-Tröpfchen eine erste innere Phase, die das erste Fluid umfasst, eine zweite innere Phase, die das zweite Fluid umfasst, und eine äußere Phase umfassen, die das dritte Fluid umfasst, wobei die erste innere Phase und die zweite innere Phase von der äußeren Phase umgeben sind; und
Strömenlassen der Dreiphasen-Tröpfchen von der siebten Austrittsöffnung zu einem Aushärtegefäß; und
Aushärten der Dreiphasen-Tröpfchen, um Dreiphasen-Mikrokugeln zu bilden,
wobei:
das erste Fluid ein Gas oder ein ungehärtetes erstes Harz umfasst;
das zweite Fluid ein ungehärtetes zweites Harz umfasst;
mindestens eines aus dem ersten Fluid, dem zweiten Fluid, oder beide, ein radioaktives Isotop oder eine Verbindung umfassen, die bei Neutronenbestrahlung der Verbindung radioaktiv gemacht werden kann;
das dritte Fluid ein ungehärtetes drittes Harz umfasst; und
das vierte Fluid mit dem dritten Fluid unmischbar ist.

10. Verfahren nach Anspruch 8 oder 9, wobei es sich bei dem ersten Harz um ein biologisch resorbierbares Harz oder ein biologisch abbaubares Harz handelt; und/oder wobei es sich bei dem ersten Harz um ein wasserquellbares Polymer handelt; und/oder wobei das erste ungehärtete Harz und das zweite ungehärtete Harz identisch sind.

11. Verfahren nach Anspruch 8 bis 10, wobei das radioaktive Isotop ein beta-emittierendes Isotop oder ein gamma-emittierendes Isotop umfasst.

12. Verfahren nach einem der Ansprüche 8 bis 11, wobei das erste Fluid das ungehärtete erste Harz und eine Verbindung umfasst, die bei Neutronenbestrahlung der Verbindung radioaktiv gemacht werden kann, wobei das Verfahren weiter das Bestrahlen der Zweiphasen-Mikrokugeln mit Neutronen umfasst, gegebenenfalls wobei die Verbindung Yttrium-89 umfasst und die Neutronenbestrahlung das Yttrium-89 in Yttrium-90 umwandelt.

13. Verfahren nach einem der Ansprüche 8 bis 12, wobei das erste Fluid, das zweite Fluid, oder beide, weiter ein therapeutisches Mittel umfassen; und/oder
wobei das erste Fluid, das zweite Fluid, oder beide, weiter einen Fluoreszenzfarbstoff umfassen.

14. Verfahren nach einem der Ansprüche 9 bis 13, wobei es sich bei dem ersten Fluid um ein Gas handelt, wobei es sich gegebenenfalls bei dem Gas um Luft handelt.

15. Verfahren nach einem der Ansprüche 8 bis 14, wobei das radioaktive Isotop Yttrium-90 umfasst.

## Revendications

1. Microsphère multiphase pour radio-embolisation, la microsphère multiphase comprenant :
une phase primaire comprenant une première résine ; et
une première phase secondaire entourée par la phase primaire, la première phase secondaire comprenant une deuxième résine et au moins un parmi un isotope radioactif ou un composé comprenant au moins un élément radioactif.

2. Microsphère multiphase selon la revendication 1, dans laquelle la première résine est une résine biorésorbable ou une résine biodégradable ; et/ou dans laquelle la première résine est un polymère gonflable dans l'eau ; et/ou dans laquelle la première résine et la deuxième résine sont identiques.

3. Microsphère multiphase selon l'une quelconque des revendications précédentes, dans laquelle l'isotope radioactif comprend un isotope émettant un rayonnement bêta ou un isotope émettant un rayonnement gamma.

4. Microsphère multiphase selon l'une quelconque des revendications précédentes, dans laquelle la microsphère multiphase comprend en outre un agent thérapeutique ; et/ou
dans laquelle la phase primaire, la phase secondaire, ou les deux, comprennent en outre un colorant fluorescent.

5. Microsphère multiphase selon l'une quelconque des revendications précédentes, dans laquelle la première résine est stable à un rayonnement gamma ; et/ou dans laquelle la première résine est imperméable à l'eau.

6. Microsphère multiphase selon l'une quelconque des revendications précédentes, comprenant en outre une deuxième phase secondaire entourée par la phase primaire, la deuxième phase secondaire comprenant un gaz, éventuellement dans laquelle le gaz est de l'air.

7. Microsphère multiphase selon la revendication 6, dans laquelle la microsphère multiphase est à flottabilité neutre dans l'eau ou est à flottabilité neutre dans le sang humain.

8. Procédé de formation de microsphères en deux phases, le procédé comprenant :
l'écoulement d'un premier fluide à travers un premier conduit longitudinal vers une première ouverture de sortie du premier conduit longitudinal, la première ouverture de sortie étant en communication fluidique avec une première zone de contact ;
l'écoulement d'un deuxième fluide à travers un premier conduit transversal, le premier conduit transversal croisant le premier conduit longitudinal au niveau de la première zone de contact et étant en communication fluidique avec la première ouverture de sortie et avec une deuxième ouverture de sortie du premier conduit transversal, la deuxième ouverture de sortie étant en communication fluidique avec un deuxième conduit longitudinal, selon lequel un flux biphasique s'écoule dans le deuxième conduit longitudinal vers une troisième ouverture de sortie du deuxième conduit longitudinal, la troisième ouverture de sortie étant en communication fluidique avec une deuxième zone de contact, le flux biphasique comprenant des gouttelettes du premier fluide entourées par une phase continue du deuxième fluide ;
l'écoulement d'un troisième fluide à travers un deuxième conduit transversal, le deuxième conduit transversal croisant le deuxième conduit longitudinal au niveau de la deuxième zone de contact et étant en communication fluidique avec la troisième ouverture de sortie et avec une quatrième ouverture de sortie du deuxième conduit transversal, la quatrième ouverture de sortie étant en communication fluidique avec un troisième conduit longitudinal, selon lequel un flux triphasique s'écoule dans le troisième conduit longitudinal vers une cinquième ouverture de sortie du troisième conduit longitudinal, le flux triphasique comprenant des gouttelettes en deux phases entourées par une phase continue du troisième fluide, les gouttelettes en deux phases comprenant une phase interne comprenant le premier fluide entourée par une phase externe comprenant le deuxième fluide ; et
l'écoulement des gouttelettes en deux phases de la cinquième ouverture de sortie à un récipient de durcissement ; et
le durcissement des gouttelettes en deux phases pour former des microsphères en deux phases,
dans lequel :
le premier fluide comprend une première résine non durcie et au moins un parmi un isotope radioactif ou un composé qui peut être rendu radioactif lors d'une irradiation neutronique du composé ;
le deuxième fluide comprend une deuxième résine non durcie ;
le troisième fluide est non miscible avec le deuxième fluide.

9. Procédé de formation de microsphères en trois phases, le procédé comprenant :
l'écoulement d'un premier fluide à travers un premier conduit longitudinal vers une première ouverture de sortie du premier conduit longitudinal, la première ouverture de sortie étant en communication fluidique avec une première zone de contact ;
l'écoulement d'un deuxième fluide à travers un premier conduit transversal, le premier conduit transversal croisant le premier conduit longitudinal au niveau de la première zone de contact et étant en communication fluidique avec la première ouverture de sortie et avec une deuxième ouverture de sortie du premier conduit transversal, la deuxième ouverture de sortie étant en communication fluidique avec un deuxième conduit longitudinal, selon lequel un flux biphasique s'écoule dans le deuxième conduit longitudinal vers une troisième ouverture de sortie du deuxième conduit longitudinal, la troisième ouverture de sortie étant en communication fluidique avec une deuxième zone de contact, le flux biphasique comprenant des gouttelettes du premier fluide entourées par une phase continue du deuxième fluide ;
l'écoulement d'un troisième fluide à travers un deuxième conduit transversal, le deuxième conduit transversal croisant le deuxième conduit longitudinal au niveau de la deuxième zone de contact et étant en communication fluidique avec la troisième ouverture de sortie et avec une quatrième ouverture de sortie du deuxième conduit transversal, la quatrième ouverture de sortie étant en communication fluidique avec un troisième conduit longitudinal, selon lequel un flux triphasique s'écoule dans le troisième conduit longitudinal vers une cinquième ouverture de sortie du troisième conduit longitudinal, la cinquième ouverture de sortie étant en communication fluidique avec une troisième zone de contact, le flux triphasique comprenant des gouttelettes en deux phases entourées par une phase continue comprenant le troisième fluide, les gouttelettes en deux phases comprenant une phase interne comprenant le premier fluide entourée par une phase externe comprenant le deuxième fluide ; et
l'écoulement d'un quatrième fluide à travers un troisième conduit transversal, le troisième conduit transversal croisant le troisième conduit longitudinal au niveau de la troisième zone de contact et étant en communication fluidique avec la cinquième ouverture de sortie et avec une sixième ouverture de sortie du troisième conduit transversal, la sixième ouverture de sortie étant en communication fluidique avec un quatrième conduit longitudinal, selon lequel un flux tétraphasique s'écoule dans le quatrième conduit longitudinal vers une septième ouverture de sortie du quatrième conduit longitudinal, le flux tétraphasique comprenant des gouttelettes en trois phases entourées par une phase continue du quatrième fluide, les gouttelettes en trois phases comprenant une première phase interne comprenant le premier fluide, une deuxième phase interne comprenant le deuxième fluide, et une phase externe comprenant le troisième fluide, la première phase interne et la deuxième phase interne étant entourées par la phase externe ; et
l'écoulement des gouttelettes en trois phases de la septième ouverture de sortie à un récipient de durcissement ; et
le durcissement des gouttelettes en trois phases pour former des microsphères en trois phases,
dans lequel :
le premier fluide comprend un gaz ou une première résine non durcie ;
le deuxième fluide comprend une deuxième résine non durcie ;
au moins un parmi le premier fluide, le deuxième fluide, ou les deux, comprend un isotope radioactif ou un composé qui peut être rendu radioactif lors d'une irradiation neutronique du composé ;
le troisième fluide comprend une troisième résine non durcie ; et
le quatrième fluide est non miscible avec le troisième fluide.

10. Procédé selon la revendication 8 ou 9, dans lequel la première résine est une résine biorésorbable ou une résine biodégradable ; et/ou dans lequel la première résine est un polymère gonflable dans l'eau ; et/ou dans lequel la première résine non durcie et la deuxième résine non durcie sont identiques.

11. Procédé selon les revendications 8 à 10, dans lequel l'isotope radioactif comprend un isotope émettant un rayonnement bêta ou un isotope émettant un rayonnement gamma.

12. Procédé selon l'une quelconque des revendications 8 à 11, dans lequel le premier fluide comprend la première résine non durcie et un composé qui peut être rendu radioactif lors d'une irradiation neutronique du composé, le procédé comprenant en outre l'irradiation neutronique des microsphères en deux phases, éventuellement dans lequel le composé comprend de l'yttrium-89 et l'irradiation neutronique convertit l'yttrium-89 en yttrium-90.

13. Procédé selon l'une quelconque des revendications 8 à 12, dans lequel le premier fluide, le deuxième fluide, ou les deux, comprennent en outre un agent thérapeutique ; et/ou
dans lequel le premier fluide, le deuxième fluide, ou les deux, comprennent en outre un colorant fluorescent.

14. Procédé selon l'une quelconque des revendications 9 à 13, dans lequel le premier fluide est un gaz, éventuellement dans lequel gaz est de l'air.

15. Procédé selon l'une quelconque des revendications 8 à 14, dans lequel l'isotope radioactif comprend de l'yttrium-90.
